(19) Europäisches Patentamt
European Patent Office
Office européen des brevets

(11) Numéro de publication : **0 445 039 B1**

# (12) FASCICULE DE BREVET EUROPEEN

(45) Date de publication du fascicule du brevet : **08.06.94 Bulletin 94/23**

(51) Int. Cl.$^5$ : **C07D 213/74,** C07D 401/14, A61K 31/44

(21) Numéro de dépôt : **91400563.2**

(22) Date de dépôt : **01.03.91**

## (54) Nouveaux dérivés de la pyridylsulfonylurée et de la pyridylsulfonylthiourée leur procédé de préparation et les compositions pharmaceutiques que les contiennent.

(30) Priorité : **02.03.90 FR 9002659**

(43) Date de publication de la demande : **04.09.91 Bulletin 91/36**

(45) Mention de la délivrance du brevet : **08.06.94 Bulletin 94/23**

(84) Etats contractants désignés : **AT BE CH DE DK ES FR GB GR IT LI LU NL SE**

(56) Documents cités :
**EP-A- 0 003 383**
**FR-A- 2 267 775**

(73) Titulaire : **ADIR ET COMPAGNIE**
**1 rue Carle Hébert**
**F-92415 Courbevoie Cédex (FR)**

(72) Inventeur : **Masereel, Bernard**
**70 rue des vieux fours**
**B-6910 Libin (BE)**
Inventeur : **Pirotte, Bernard**
**5 rue Tollet**
**B-4480 Oupeye (BE)**
Inventeur : **Schynts, Marc**
**43 rue Général Modard**
**B-4431 Loncin (BE)**
Inventeur : **Delarge, Jacques**
**7 rue Haie des chènes**
**B-4052 Dolembreux (BE)**

EP 0 445 039 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de la pyridylsulfonylurée et de la pyridylsulfonylthiourée, leur procédé de préparation et les préparations pharmaceutiques qui les contiennent.

On connait dans la littérature de nombreuses sulfonylurées ou sulfonylthiourées, dotées notamment de propriétés diurétiques ou hypoglycémiantes. On connait également des pyridylsulfonylurées comme le Torasémide qui est un diurétique puissant.

D'autres pyridylsulfonylurées ont également été décrites pour leur propriétés diurétiques et antiinflammatoires (Brevets Français N° 2 267 775 et N° 2 416 225).

Leur effet diurétique est dû à une action inhibitrice du cotransport $Na^+$ $K^+$ $2Cl^-$ au niveau rénal.

La demanderesse a maintenant découvert de nouvelles pyridylsulfonylurées et pyridylsulfonylthiourées dotées non seulement de propriétés inhibitrices du cotransport $Na^+$ $K^+$ $2Cl^-$ et d'un canal chlorure rénal, mais également de propriétés bloquantes du canal potassique cellulaire au niveau rénal et pulmonaire.

Ces nouveaux dérivés, contrairement aux dérivés de l'art antérieur, ont également la propriété particulièrement intéressante de présenter un coefficient de partage et un pKa optimaux pour le passage de la barrière hémoméningée.

Les composés de l'invention sont donc particulièrement intéressants dans le traitement de maladies comme l'hypertension artérielle, les états oedémateux de toute étiologie, y compris l'oedème cérébral.

Plus spécifiquement, l'invention concerne les dérivés de formule (I)

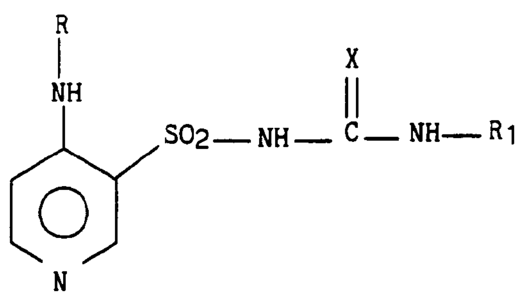

(I)

dans laquelle :

- R représente un radical cycloalkyle, bicycloalkyle ou polycycloalkyle de 3 à 15 atomes de carbone éventuellement interrompus par un ou plusieurs hétéroatomes choisis parmi azote, oxygène et soufre,
- X représente un atome d'oxygène ou de soufre.
- $R_1$ représente un radical alkyle comportant de 1 à 6 atomes de carbone en chaîne droite ou ramifiée ou un radical cycloalkyle, bicycloalkyle ou polycycloalkyle renfermant de 3 à 15 atomes de carbone, éventuellement interrompus par un ou plusieurs hétéroatomes choisis parmi azote, oxygène et soufre,
  avec la réserve que, lorsque X est un atome d'oxygène et que simultanément R est un radical alkyle monocyclique ou bicyclique, $R_1$ ne peut pas représenter un radical alkyle linéaire ou ramifié ou un groupe hétéromonocyclique azoté saturé pouvant contenir un deuxième hétéroatome,
  ainsi que leurs sels d'addition à un acide minéral ou organique pharmaceutiquement acceptable,
  et, lorsque R ou $R_1$ représentent un radical comportant un ou plusieurs centres d'asymétrie, leurs isomères, énantiomères et diastéréoisomères.

L'invention s'étend aussi au procédé de préparation des dérivés de formule (I), caractérisé en ce que :

- soit l'on condense un dérivé de formule (II)

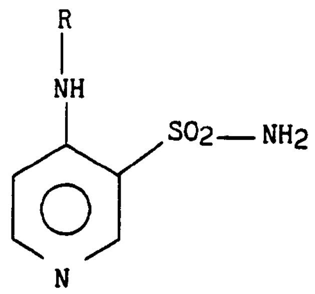

(II)

dans laquelle R a la même signification que dans la formule (I), avec un isocyanate, ou isothiocyanate de formule (III)

$$X = C = N - R_1 \quad \textbf{(III)}$$

dans laquelle X et $R_1$ ont la même signification que dans la formule (I),
en présence d'un agent alcalin tel qu'un hydroxyde métallique ou une amine tertiaire dans un solvant polaire, pour conduire, après neutralisation du milieu réactionnel précédée si l'on désire de l'évaporation du solvant et reprise par l'eau, aux dérivés de formule (I) qui sont éventuellement salifiés par addition d'un acide minéral ou organique pharmaceutiquement acceptable,
ou si l'on désire séparés en leurs isomères puis éventuellement salifiés par addition d'un acide minéral ou organique pharmaceutiquement acceptable,

- soit l'on condense un dérivé de formule (IV)

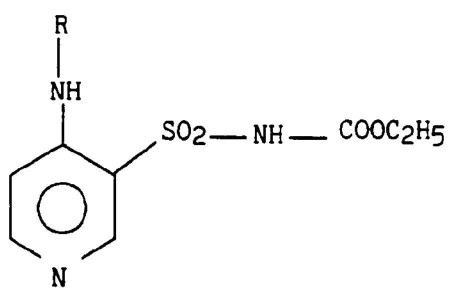

(IV)

dans laquelle R a la même signification que dans la formule (I) avec une amine de formule (V)

$$R_1 - NH_2 \quad \textbf{(V)}$$

dans laquelle $R_1$ a la même signification que dans la formule (I),
par chauffage dans un solvant anhydre pour obtenir, après purification et neutralisation éventuelles, les composés de formule (I) dans lesquels X représente un atome d'oxygène,
qui sont ensuite éventuellement salifiés par un acide minéral ou organique pharmaceutiquement acceptable.
ou si l'on désire séparés en leurs isomères puis éventuellement salifiés par addition d'un acide minéral ou organique pharmaceutiquement acceptable.

Parmi les acides minéraux ou organiques pharmaceutiquement acceptables l'on peut citer, sans que cette liste soit limitative, les acides chlorhydrique, sulfurique, nitrique, acétique, tartrique, malique, maléïque, camphorique, méthanesulfonique, éthanesulfonique, camphosulfonique,...

Les composés de l'invention possèdent des propriétés pharmacologiques trés intéressantes.

Ils présentent des propriétés diurétiques dues à l'inhibition du cotransport $Na^+ K^+ 2Cl^-$ et d'un canal chlorure, doublées de propriétés d'épargne du potassium liée à l'inhibition du canal potassique membranaire.

Les composés de l'invention trouvent donc une application dans le traitement de l'hypertension, d'autant plus intéressante que cette normalisation tensionnelle n'est pas accompagnée d'une fuite potassique dont on connait les effets néfastes, notamment pour le muscle cardiaque.

Par ailleurs, les composés de l'invention possèdent une lipophilie particulièrement bien adaptée au passage de la barrière hémoméningée et, dès lors, leur action inhibitrice du cotransport $Na^+ K^+ 2Cl^-$ peut s'exercer directement au niveau des astrocytes, empéchant ainsi le gonflement astrocytaire qui représente une part très importante de l'oedème cérébral.

Les composés de l'invention trouvent donc une application dans le traitement des états oedémateux et notamment de l'oedème cérébral.

La présente invention a également pour objet les compositions pharmaceutiques renfermant comme principe actif au moins un composé de formule (I) ou un de ses sels d'addition à un acide pharmaceutiquement acceptable, seul ou en combinaison avec un ou plusieurs excipients ou véhicules inertes atoxiques.

Parmi les compositions pharmaceutiques selon l'invention, on pourra citer plus particulièrement celles qui conviennent à l'administration orale, parentérale, nasale, rectale ou cutanée, notamment les comprimés simples ou dragéifiés, les gélules, les cachets et sachets, les comprimés sublinguaux et les glossettes, les suppositoires, les crèmes et gels dermiques, les aérosols, les solutés injectables, les gouttes nasales.

La posologie utile varie selon l'âge, le poids du patient, la sévérité de l'affection ainsi que la voie d'administration. D'une manière générale, la posologie unitaire s'échelonne entre 0,1 mg à 500 mg en une à trois prises quotidiennes.

Les exemples suivants illustrent l'invention.

Les composés de départ de formule génarale (II) et (IV) sont décrits dans la littérature (Eur. J. Med. Chem. 15 (4), 299-304 (1980) et 16 (1) 65-68 (1981)) ou peuvent être préparés de manière analogue.

Les composés de formule (II) peuvent notamment être obtenus par action d'un excès d'une amine de formule $RNH_2$ où R à la même signification que dans la formule générale (I) sur un dérivé pyridinique de formule

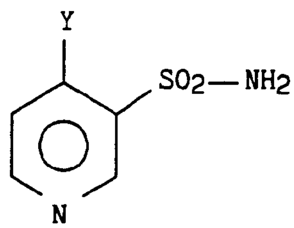

dans laquelle Y représente un groupe partant tel que halogène ou $SO_3H$, par chauffage entre 80 et 140°C. Après dilution et alcalinisation éventuelles, puis si l'on désire une clarification au charbon les composés de formule (II) sont précipités à pH 7-8, essorés, séchés, et utilisés tels quels dans la suite des réactions.

Dans les exemples qui suivent, les analyses centésimales, les spectres infra-rouge et $^1$H RMN sont en accord avec les structures attendues.

**EXEMPLE 1 :**

**N-((4-(cyclohexylamino)-pyrid-3-yl)sulfonyl)-N'-cyclohexylurée**

Une solution de 0,01 mole de (4-(cyclohexylamino)pyrid-3-yl)sulfonamide dans 80 ml du mélange eau-acétone (1:1) est additionnée de 0,01 mole de NaOH en solution dans un minimum d'eau. On agite avec un barreau magnétique et on ajoute 0,015 mole de cyclohexylisocyanate. On maintient sous agitation en suivant l'évolution de la réaction par c.c.m. (silicagel 60 F254, phase mobile: acétate d'éthyle 9, méthanol 1, triéthylamine 0,2). Un léger chauffage peut accélérer la réaction. On évapore sous dépression et le résidu est repris par 100 ml de NaOH 0,2 N. L'insoluble éventuel est filtré et la solution est amenée à pH 6,5-7 et abandonnée à cristallisation pendant 1 heure au réfrigérateur. Le précipité recueilli est mis en suspension dans 100 ml du mélange eau-acétone (4:1) saturé par $NaHCO_3$. Le mélange est agité pendant 1 heure, filtré et le filtrat ramené à pH 6,5-7 est abandonné à cristallisation au réfrigérateur. Les cristaux sont recueillis, lavés à l'eau froide et séchés sous vide à température ordinaire. Le rendement est voisin de 50 à 60 %.
point de fusion : 165-167 °C.

**EXEMPLE 2 :**

**N-((4-(cycloheptylamino)pyrid-3-yl)sulfonyl)-N'-cyclohexylurée**

0,01 mole de N-(4-(cycloheptylamino)pyrid-3-yl)sulfonylcarbamate d'éthyle, 0,05 mole de cyclohexylamine et 3 g de tamis moléculaire de 4 Å sont additionnés de 40 ml de toluène anhydre et portés à reflux pendant quelques heures en suivant l'évolution de la réaction par c.c.m. (silicagel 60F254 phase mobile : acétate d'éthyle 9, méthanol 1, acide acétique 0,2). On sépare le tamis moléculaire et on évapore sous dépression. Le résidu est repris par 100 à 150 ml de NaOH 0,2 N et extrait 2 fois par 50 ml d'éther. La phase aqueuse est

amenée à pH 6,5-7 et abandonnée à cristallisation pendant 1 heure au réfrigérateur. Les opérations sont alors poursuivies comme dans l'exemple 1). Le rendement est voisin de 50 à 60 %.
Point de fusion : 165-169 °C.

**EXEMPLE 3 :**

**Nitrate de N-((4-(cyclooctylamino)pyrid-3-yl)sulfonyl)-N′-cyclooctylurée**

On utilise les conditions opératoires de l'exemple 2 mais en employant du N-(4-(cyclooctylamino)pyrid-3-yl)sulfonylcarbamate d'éthyle et de la cyclooctylamine comme matières premières. Après séparation du tamis moléculaire et évaporation sous dépression on reprend par 75 à 100 ml de NaOH 0,2 N, on extrait deux fois par 50 ml d'éther et la phase aqueuse est additionnée de 5 à 10 ml d'acide nitrique. On abandonne une nuit au réfrigérateur.
Les cristaux sont recueillis sur filtre, lavés avec un minimum d'eau glacée et séchés sous vide à température ordinaire. Le rendement est voisin de 60 à 70 %.
Point de fusion : 144-146 °C.

**EXEMPLE 4 :**

**N-((4-(cycloheptylamino)pyrid-3-yl)sulfonyl)-N′-cyclohexylthiourée.**

Une solution de 0,01 mole de (4-(cycloheptylamino)pyrid-3-yl)sulfonamide dans 80 ml du mélange eau-acétone (1:1) est additionnée de 0,01 mole de NaOH en solution dans un minimum d'eau. On agite avec un barreau magnétique et on ajoute 0,015 mole de cyclohexylisothiocyanate. On maintient sous agitation en suivant l'évolution de la réaction par c.c.m. (silicagel 60F254, phase mobile : acétate d'éthyle 9, méthanol 1, triéthylamine 0,2). Un léger chauffage peut accélérer la réaction. On évapore sous dépression et le résidu est repris par 100 ml de NaOH 0,2 N. L'insoluble éventuel est filtré et la solution est amenée à pH 6,5-7 et abandonnée à cristallisation pendant 1 heure au réfrigérateur. Le précipité recueilli est mis en suspension dans 100 ml du mélange eau-acétone (4:1) saturé par $NaHCO_3$. Le mélange est agité pendant 1 heure, filtré et le filtrat ramené à pH 6,5-7 est abandonné à cristallisation au réfrigérateur. Les cristaux sont recueillis, lavés à l'eau froide et séchés sous vide à température ordinaire. Le rendement est voisin de 50 à 60 %.
Point de fusion : 195-197 °C.

**EXEMPLE 5 :**

**N-((4-(norbornylamino)pyrid-3-yl)sulfonyl)-N′-isopropylthiourée**

2 grammes de (4-(norbornylamino)pyrid-3-yl)sulfonamide sont dissous dans 20 ml d'acétone et additionnés de 5 ml d'isopropylisothiocyanate et de 5 ml de triéthylamine. On chauffe à reflux en suivant l'évolution de la réaction par c.c.m. (silicagel 60F254, phase mobile : acétate d'éthyle 9, méthanol 1, triéthylamine 0,2). On évapore à sec, on reprend par 120 ml de NaOH 0,2 N et on filtre. On extrait 3 fois par 150 ml d'éther, on clarifie au charbon et on amène à pH 7,5. Le produit cristallise. Le rendement est voisin de 50 %. Point de fusion : 194-196 °C.

**EXEMPLE 6 :**

**N-((4-(cyclooctylamino)pyrid-3-yl)sulfonyl)-N′-éthylthiourée.**

Le mode opératoire est identique à celui de l'exemple 5 en utilisant le (4-(cyclooctylamino)pyrid-3-yl)sulfonamide et l'éthylisothiocyanate comme matières premières. Le rendement est similaire. Point de fusion : 196-198 °C.
En utilisant les modes opératoires décrits dans les exemples précédents, on obtient de la même façon les dérivés des exemples 7 à 17.

**EXEMPLE 7 :**

**N-((4-(cyclohexylamino)pyrid-3-yl)sulfonyl)-N′-cyclooctylurée**
Point de fusion : 133-137 °C

**EXEMPLE 8 :**

**N-((4-(cyclooctylamino)pyrid-3-yl)sulfonyl)-N′-cycloheptylurée**

Point de fusion : 156-160 °C

**EXEMPLE 9 :**

**N-((4-(cyclohexylamino)pyrid-3-yl)sulfonyl)-N′-cycloheptylurée**
Point de fusion : 145-150 °C

**EXEMPLE 10 :**

**N-((4-(cyclooctylamino)pyrid-3-yl)sulfonyl)-N′-cyclohexylurée**
Point de fusion : 144-148 °C

**EXEMPLE 11 :**

**N-((4-(cycloheptylamino)pyrid-3-yl)sulfonyl)-N′-cyclooctylurée**
Point de fusion : 140-145 °C

**EXEMPLE 12 :**

**N-((4-(cyclododécylamino)pyrid-3-yl)sulfonyl)-N′-éthylurée**
Point de fusion : 195-196 °C

**EXEMPLE 13 :**

**N-((4-(cyclododécylamino)pyrid-3-yl)sulfonyl)-N′-isopropylurée**
Point de fusion : 126-128 °C

**EXEMPLE 14 :**

**N-((4-(cycloheptylamino)pyrid-3-yl)sulfonyl)-N′-isopropylthiourée**
Point de fusion : 196-198 °C

**EXEMPLE 15 :**

**N-((4-(cyclooctylamino)pyrid-3-yl)sulfonyl)-N′-isopropylthiourée**
Point de fusion : 194-195 °C

**EXEMPLE 16 :**

**N-((4-(cyclohexylamino)pyrid-3-yl)sulfonyl)-N′-éthylthiourée**
Point de fusion : 186-187 °C

**EXEMPLE 17 :**

**N-((4-(2-(aza-2-bicyclo [3.3.0]octyl)-amino)pyrid-3-yl)sulfonyl)-N′-isopropylurée**
Point de fusion : 185-186 °C

**EXEMPLE 18 :**

**Détermination des coefficients de partage et des pKa**

Les coefficients de partage entre n-octanol et eau ont été déterminés à pH = 7,4 selon les techniques classiques et sont exprimés sous forme de logarithme décimal (Log P)

Les pKa représentent la constante d'acidité du proton $SO_2$-NH-CO-

| EXEMPLES | Log P | pKa |
|---|---|---|
| Torasemide | 0,449 | 6,82 |
| Ex 1 | 1,331 | 9,02 |
| Ex 2 | 1,665 | 9,39 |
| Ex 3 | 2,704 | 7,74 |
| Ex 7 | 2,074 | 9,13 |
| Ex 8 | 2,449 | 9,15 |
| Ex 10 | 2,063 | 8,95 |

Ces résultats indiquent une lipophilie au pH physiologique en faveur du passage de la barrière hémoméningée.

**EXEMPLE 19 :**

**Inhibition du cotransport $Na^+$ $K^+$ 2 $Cl^-$ et du canal $Cl^-$**

La concentration inhibant à 50 % le cotransport $Na^+$ $K^+$ 2 $Cl^-$ présent chez l'animal est réduite, avec le composé de l'exemple 3, d'environ 60 % par rapport au Torasémide (membrane luminale de la branche ascendante de l'anse de Henlé du néphron de lapin perfusé (Arzneim. Forsch. (1988) 38 (1a), 151-152)) et de 85 % par rapport au Torasémide avec le composé de l'exemple 10 (membrane de globules rouges de rat normotendu).

La concentration inhibant à 50 % le canal $Cl^-$ au niveau rénal chez l'animal est, avec le composé de l'exemple 8, réduite d'environ 50 % par rapport au Torasémide.

**EXEMPLE 20 :**

**Comprimés dosés à 5 mg de N-((4-(cyclohexylamino)pyrid-3-yl)sulfonyl)-N'-cyclohexylurée**

Formule pour 10 000 comprimés

| | | |
|---|---|---|
| N-((4(cyclohexylamino)pyrid-3yl)sulfonyl N'-cyclohexylurée | 50 | grammes |
| Lactose | 150 | grammes |
| Amidon de maïs | 750 | grammes |
| Silice colloïdale | 2 | grammes |
| Stéarate de magnésium | 1 | gramme |

## Revendications

**Revendications pour les Etats contractants suivants : AT, BE, CH, DE, DK, FR, GB, IT, LI, LU, NL, SE**

**1.** Dérivés de formule (I)

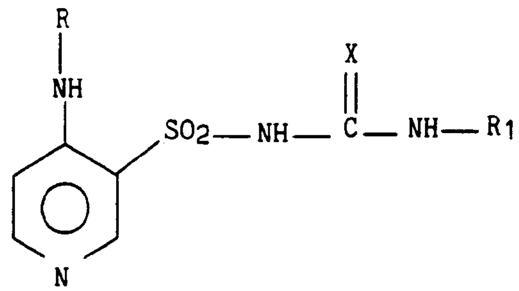

(I)

dans laquelle :

- R représente un radical cycloalkyle, bicycloalkyle ou polycycloalkyle de 3 à 15 atomes de carbone éventuellement interrompus par un ou plusieurs hétéroatomes choisis parmi azote, oxygène et soufre,
- X représente un atome d'oxygène ou de soufre.
- $R_1$ représente un radical alkyle comportant de 1 à 6 atomes de carbone en chaîne droite ou ramifiée ou un radical cycloalkyle, bicycloalkyle ou polycycloalkyle renfermant de 3 à 15 atomes de carbone, éventuellement interrompus par un ou plusieurs hétéroatomes choisis parmi azote, oxygène et soufre,

  avec la réserve que, lorsque X est un atome d'oxygène et que simultanément R est un radical alkyle monocyclique ou bicyclique, $R_1$ ne peut pas représenter un radical alkyle linéaire ou ramifié ou un groupe hétéromonocyclique azoté saturé pouvant contenir un deuxième hétéroatome,

ainsi que leurs sels d'addition à un acide minéral ou organique pharmaceutiquement acceptable, et, lorsque R ou $R_1$ représentent un radical comportant un ou plusieurs centres d'asymétrie, leurs isomères, énantiomères et diastéréoisomères.

**2.** Composés selon la revendication 1 tels que R et $R_1$ représentent un radical cycloalkyle, bicycloalkyle ou polycycloalkyle de à 3 à 15 atomes de carbone éventuellement interrompus par un ou plusieurs atomes d'azote, ainsi que leurs sels, énantiomères et diastéréoisomères.

**3.** La N-(4-(cyclohexylamino)-pyrid-3-yl)sulfonyl)-N'-cyclohexylurée et ses sels.

**4.** La N-((4-(cycloheptylamino)pyrid-3-yl)sulfonyl)-N'-cyclohexylurée et ses sels.

**5.** La N-((4-(cyclooctylamino)pyrid-3-yl)sulfonyl)-N'-cyclooctylurée et ses sels.

**6.** La N-((4-(cycloheptylamino)pyrid-3-yl)sulfonyl)-N'-cyclohexylthiourée et ses sels.

**7.** La N-((4-(norbornylamino)pyrid-3-yl)sulfonyl)-N'-isopropylthiourée et ses sels.

**8.** La N-((4-(cyclooctylamino)pyrid-3-yl)sulfonyl)-N'-éthylthiourée et ses sels.

**9.** La N-((4-(cyclododécylamino)pyrid-3-yl)sulfonyl)-N'-éthylurée et ses sels.

**10.** La N-((4-(2-(aza-2-bicyclo [3.3.0]octyl)-amino)pyrid-3-yl)sulfonyl)-N'-isopropylurée, ses sels et ses diastéréoisomères.

**11.** Procédé de préparation des dérivés de formule (I), caractérisé en ce que :

- soit l'on condense un dérivé de formule (II)

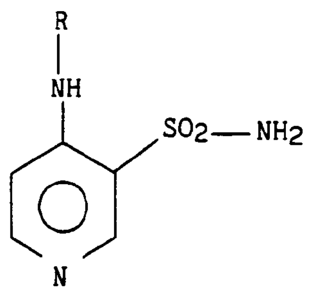

(II)

dans laquelle R a la même signification que dans la formule (I), avec un isocyanate, ou isothiocyanate de formule (III)

$$X = C = N - R_1 \quad \textbf{(III)}$$

dans laquelle X et $R_1$ ont la même signification que dans la formule (I),
en présence d'un agent alcalin tel qu'un hydroxyde métallique ou une amine tertiaire dans un solvant polaire, pour conduire, après neutralisation du milieu réactionnel précédée si l'on désire de l'évaporation du solvant et reprise par l'eau, aux dérivés de formule (I) qui sont éventuellement salifiés, par addition d'un acide minéral ou organique pharmaceutiquement acceptable,
ou si l'on désire séparés en leurs isomères puis éventuellement salifiés par addition d'un acide minéral ou organique pharmaceutiquement acceptable,

- soit l'on condense un dérivé de formule (IV)

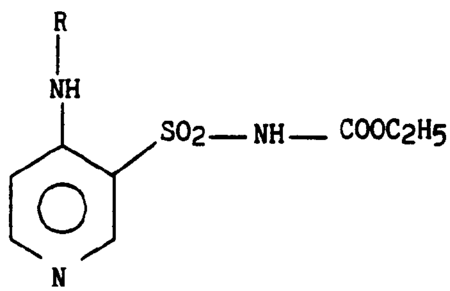

(IV)

dans laquelle R a la même signification que dans la formule (I) avec une amine de formule (V)

$$R_1 - NH_2 \quad \textbf{(V)}$$

dans laquelle $R_1$ a la même signification que dans la formule (I),
par chauffage dans un solvant anhydre pour obtenir, après purification et neutralisation éventuelles, les composés de formule (I) dans lesquels X représente un atome d'oxygène,
qui sont ensuite éventuellement salifiés par un acide minéral ou organique pharmaceutiquement acceptable,
ou si l'on désire séparés en leurs isomères puis éventuellement salifiés par addition d'un acide minéral ou organique pharmaceutiquement acceptable.

**12.** Compositions pharmaceutiques contenant comme principe actif au moins un composé selon l'une quelconque des revendications 1 à 10, seul ou en combinaison avec un ou plusieurs excipients inertes non toxiques.

**13.** Compositions pharmaceutiques selon la revendication 12 utiles dans le traitement de l'hypertension artérielle et des états oedémateux.

**Revendications pour les Etats contractants suivants : ES, GR**

**1.** Procédé de préparation des dérivés de formule (I)

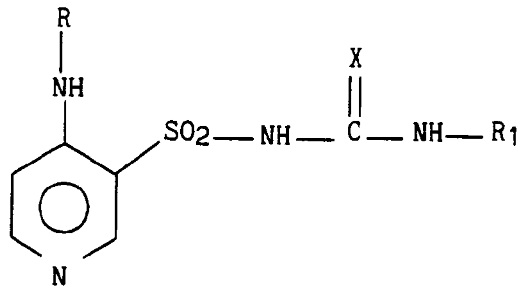

(I)

dans laquelle :

- R représente un radical cycloalkyle, bicycloalkyle ou polycycloalkyle de 3 à 15 atomes de carbone éventuellement interrompus par un ou plusieurs hétéroatomes choisis parmi azote, oxygène et soufre,
- X représente un atome d'oxygène ou de soufre
- $R_1$ représente un radical alkyle comportant de 1 à 6 atomes de carbone en chaîne droite ou ramifiée ou un radical cycloalkyle, bicycloalkyle ou polycycloalkyle renfermant de 3 à 15 atomes de carbone, éventuellement interrompus par un ou plusieurs hétéroatomes choisis parmi azote, oxygène et soufre,

  avec la réserve que, lorsque X est un atome d'oxygène et que simultanément R est un radical alkyle monocyclique ou bicyclique, $R_1$ ne peut pas représenter un radical alkyle linéaire ou ramifié ou un groupe hétéromonocyclique azoté saturé pouvant contenir un deuxième hétéroatome,

ainsi que leurs sels d'addition à un acide minéral ou organique pharmaceutiquement acceptable,
et, lorsque R ou $R_1$ représentent un radical comportant un ou plusieurs centres d'asymétrie, leurs isomères, énantiomères et diastéréoisomères,
caractérisé en ce que :

- soit l'on condense un dérivé de formule (II)

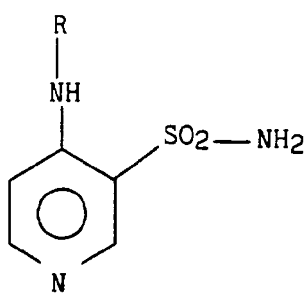

(II)

  dans laquelle R a la même signification que dans la formule (I), avec un isocyanate, ou isothiocyanate de formule (III)

  $$X = C = N - R_1 \qquad \textbf{(III)}$$

  dans laquelle X et $R_1$ ont la même signification que dans la formule (I),
  en présence d'un agent alcalin tel qu'un hydroxyde métallique ou une amine tertiaire dans un solvant polaire, pour conduire, après neutralisation du milieu réactionnel précédée si l'on désire de l'évaporation du solvant et reprise par l'eau, aux dérivés de formule (I) qui sont éventuellement salifiés, par addition d'un acide minéral ou organique pharmaceutiquement acceptable,
  ou si l'on désire séparés en leurs isomères puis éventuellement salifiés par addition d'un acide minéral ou organique pharmaceutiquement acceptable,
- soit l'on condense un dérivé de formule (IV)

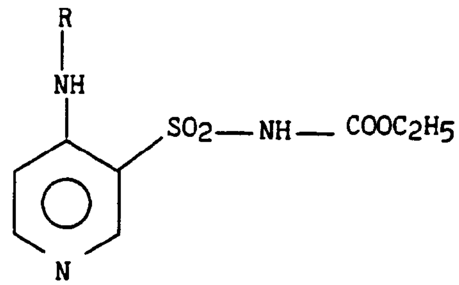

(IV)

dans laquelle R a la même signification que dans la formule (I) avec une amine de formule (V)

$R_1$ - $NH_2$ **(V)**

dans laquelle $R_1$ a la même signification que dans la formule (I),
par chauffage dans un solvant anhydre pour obtenir, après purification et neutralisation éventuelles, les composés de formule (I) dans lesquels X représente un atome d'oxygène,
qui sont ensuite éventuellement salifiés par un acide minéral ou organique pharmaceutiquement acceptable,
ou si l'on désire séparés en leurs isomères puis éventuellement salifiés par addition d'un acide minéral ou organique pharmaceutiquement acceptable.

**2.** Procédé de préparation selon la revendication 1 de composé de formule générale I dans lesquels R et $R_1$ représentent un radical cycloalkyle, bicycloalkyle ou polycycloalkyle de à 3 à 15 atomes de carbone éventuellement interrompus par un ou plusieurs atomes d'azote, ainsi que leurs sels, énantiomères et diastéréoisomères,

**3.** Procédé de préparation selon la revendication 1 de composé de formule générale 1 qui est la N-(4-(cyclohexylamino)-pyrid-3-yl)sulfonyl)-N'-cyclohexylurée et ses sels.

**4.** Procédé de préparation selon la revendication 1 de composé de formule générale 1 qui est la N-((4-(cycloheptylamino)pyrid-3-yl)sulfonyl)-N'-cyclohexylurée et ses sels.

**5.** Procédé de préparation selon la revendication 1 de composé de formule générale 1 qui est la N-((4-(cyclooctylamino)pyrid-3-yl)sulfonyl)-N'-cyclooctylurée et ses sels.

**6.** Procédé de préparation selon la revendication 1 de composé de formule générale 1 qui est la N-((4-(cycloheptylamino)pyrid-3-yl)sulfonyl)-N'-cyclohexylthiourée et ses sels.

**7.** Procédé de préparation selon la revendication 1 de composé de formule générale 1 qui est la N-((4-(norbornylamino)pyrid-3-yl)sulfonyl)-N'-isopropylthiourée et ses sels.

**8.** Procédé de préparation selon la revendication 1 de composé de formule générale 1 qui est la N-((4-(cyclooctylamino)pyrid-3-yl)sulfonyl)-N'-éthylthiourée et ses sels.

**9.** Procédé de préparation selon la revendication 1 de composé de formule générale 1 qui est la N-((4-(cyclododécylamino)pyrid-3-yl)sulfonyl)-N'-éthylurée et ses sels.

**10.** Procédé de préparation selon la revendication 1 de composé de formule générale 1 qui est la N-((4-(2-(aza-2-bicyclo [3.3.0]octyl)-amino)pyrid-3-yl)sulfonyl)-N'-isopropylurée, ses sels et ses diastéréoisomères.

**11.** Procédé de préparation de compositions pharmaceutiques contenant comme principe actif au moins un composé selon l'une quelconque des revendications 1 à 10, seul ou en combinaison avec un ou plusieurs excipients inertes non toxiques.

**12.** Procédé de préparation selon la revendication 11 de compositions pharmaceutiques selon la revendication 11 utiles dans le traitement de l'hypertension artérielle et des états oedémateux.

## Patentansprüche

**Patentansprüche für folgende Vertragsstaaten : AT, BE, CH, DE, DK, FR, GB, IT, LI, LU, NL, SE**

**1.** Derivate der Formel (I)

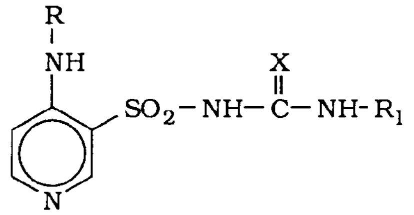

(I)

in der:
- R eine Cycloalkyl-, Bicycloalkyl- oder Polycycloalkylgruppe mit 3 bis 15 Kohlenstoffatomen, die gegebenenfalls durch eines oder mehrere Heteroatome ausgewählt aus Stickstoff. Sauerstoff und Schwefel, unterbrochen sein können,
- X ein Sauerstoffatom oder ein Schwefelatom und
- $R_1$ eine geradkettige oder verzweigte Alkylgruppe mit 1 bis 6 Kohlenstoffatomen oder eine Cycloalkyl-, Bicycloalkyl- oder Polycycloalkylgruppe mit 3 bis 15 Kohlenstoffatomen, die gegebenenfalls durch eines oder mehrere Heteroatome ausgewählt aus Stickstoff, Sauerstoff und Schwefel, unterbrochen sein können,
  mit der Maßgabe bedeuten, daß, wenn X ein Sauerstoffatom und gleichzeitig R eine monocyclische oder bicyclische Alkylgruppe bedeuten, $R_1$ keine geradkettige oder verzweigte Alkylgruppe oder eine gesättigte stickstoffhaltige heteromonocyclische Gruppe, die ein zweites Heteroatom enthalten kann, darstellt,

sowie deren Additionssalze mit einer anorganischen oder organischen, pharmazeutisch annehmbaren Säure
und, wenn R oder $R_1$ eine Gruppe bedeuten, die eines oder mehrere asymmetrische Zentren aufweisen, deren Isomere, Enantiomere und Diastereoisomere.

**2.** Verbindungen nach Anspruch 1, worin R und $R_1$ eine Cycloalkyl-, Bicycloalkyl- oder Polycycloalkylgruppe mit 3 bis 15 Kohlenstoffatomen, die gegebenenfalls durch eines oder mehrere Stickstoffatome unterbrochen sind, bedeuten, sowie deren Salze, Enantiomere und Diastereoisomere.

**3.** N-(4-(Cyclohexylamino)-pyrid-3-yl)-sulfonyl)-N′-cyclohexylharnstoff und dessen Salze.

**4.** N-((4-(Cycloheptylamino)-pyrid-3-yl)-sulfonyl)-N′-cyclohexylharnstoff und dessen Salze.

**5.** N-((4-(Cyclooctylamino)-pyrid-3-yl)-sulfonyl)-N′-cyclooctylharnstoff und dessen Salze.

**6.** N-((4-(Cycloheptylamino)-pyrid-3-yl)-sulfonyl)-N′-cyclohexylthioharnstoff und dessen Salze.

**7.** N-((4-(Norbornylamino)-pyrid-3-yl)-sulfonyl)-N′-diisopropylthioharnstoff und dessen Salze.

**8.** N-((4-(Cyclooctylamino)-pyrid-3-yl)-sulfonyl)-N′-ethylthioharnstoff und dessen Salze.

**9.** N-((4-(Cyclododecylamino)-pyrid-3-yl)-sulfonyl)-N′-ethylharnstoff und dessen Salze.

**10.** N-((4-(2-(Aza-2-bicyclo[3.3.0]octyl)-amino)-pyrid-3-yl)-sulfonyl)-N′-isopropylharnstoff, dessen Salze und dessen Diastereoisomere.

**11.** Verfahren zur Herstellung der Derivate der Formel (I), **dadurch gekennzeichnet,** daß man
- entweder ein Derivat der Formel (II)

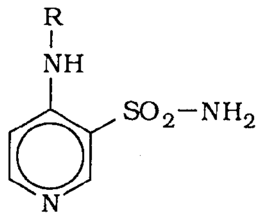

(II)

in der R die bezüglich der Formel (I) angegebenen Bedeutungen besitzt, mit einem Isocyanat oder Isothiocyanat der Formel (III)

$$X = C = N - R_1 \qquad (III)$$

in der X und $R_1$ die bezüglich der Formel (I) angegebenen Bedeutungen besitzen, in Gegenwart eines alkalischen Mittels, wie eines Metallhydroxids oder eines tertiären Amins in einem polaren Lösungsmittel kondensiert, so daß man nach der Neutralisation des Reaktionsmediums, der gewünschtenfalls ein Verdampfen des Lösungsmittels und eine Wiederaufnahme in Wasservorausgeht, die Derivate der Formel (I) erhält, die gegebenenfalls durch Zugabe einer anorganischen oder organischen, pharmazeutisch annehmbaren Säure in ihre Salze überführt werden,
oder die man gewünschtenfalls in ihre Isomeren auftrennt und gegebenenfalls durch Zugabe einer anorganischen oder organischen, pharmazeutisch annehmbaren Säure in ihre Salze überführt,

- oder ein Derivat der Formel (IV)

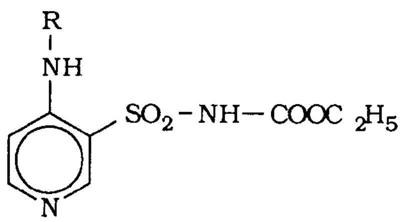

(IV)

in der R die bezüglich der Formel (I) angegebenen Bedeutungen besitzt, mit einem Amin der Formel (V)

$$R_1 - NH_2 \qquad (V)$$

in der $R_1$ die bezüglich der Formel (I) angegebenen Bedeutungen besitzt, durch Erhitzen in einem wasserfreien Lösungsmittel kondensiert, so daß man nach der eventuellen Reinigung und Neutralisation die Verbindungen der Formel (I) erhält, in der X ein Sauerstoffatom bedeutet,
die anschließend gegebenenfalls mit einer anorganischen oder organischen, pharmazeutisch annehmbaren Säure in ihre Salze überführt werden,
oder die man gewünschtenfalls in ihre Isomeren auftrennt und dann gegebenenfalls durch Zugabe einer anorganischen oder organischen, pharmazeutisch annehmbaren Säure in ihre Salze überführt.

12. Pharmazeutische Zubereitungen enthaltend als Wirkstoff mindestens eine Verbindung nach einem der Ansprüche 1 bis 10 allein oder in Kombination mit einem oder mehreren inerten, nichttoxischen Trägermaterialien.

13. Pharmazeutische Zubereitungen nach Anspruch 12 zur Behandlung der arteriellen Hypertension und von ödematösen Zuständen.

**Patentansprüche für folgende Vertragsstaaten : ES, GR**

1. Verfahren zur Herstellung der Derivate der Formel (I)

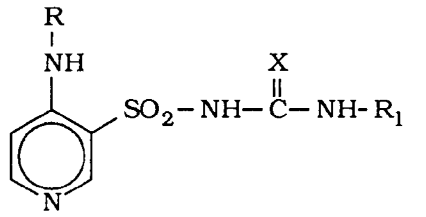

(I)

in der:

- R eine Cycloalkyl-, Bicycloalkyl- oder Polycycloalkylgruppe mit 3 bis 15 Kohlenstoffatomen, die gegebenenfalls durch eines oder mehrere Heteroatome ausgewählt aus Stickstoff, Sauerstoff und Schwefel, unterbrochen sein können,
- X ein Sauerstoffatom oder ein Schwefelatom und
- $R_1$ eine geradkettige oderverzweigte Alkylgruppe mit 1 bis 6 Kohlenstoffatomen oder eine Cycloalkyl-, Bicycloalkyl- oder Polycycloalkylgruppe mit 3 bis 15 Kohlenstoffatomen, die gegebenenfalls durch eines oder mehrere Heteroatome ausgewählt aus Stickstoff, Sauerstoff und Schwefel, unterbrochen sein können,

  mit der Maßgabe bedeuten, daß, wenn X ein Sauerstoffatom und gleichzeitig R eine monocyclische oder bicyclische Alkylgruppe bedeuten, $R_1$ keine geradkettige oder verzweigte Alkylgruppe oder eine gesättigte stickstoffhaltige heteromonocyclische Gruppe, die ein zweites Heteroatom enthalten kann, darstellt,

sowie deren Additionssalze mit einer anorganischen oder organischen, pharmazeutisch annehmbaren Säure

und, wenn R oder $R_1$ eine Gruppe bedeuten, die eines oder mehrere asymmetrische Zentren aufweisen, von deren Isomeren, Enantiomeren und Diastereoisomeren,

**dadurch gekennzeichnet,** daß man

- entweder ein Derivat der Formel (II)

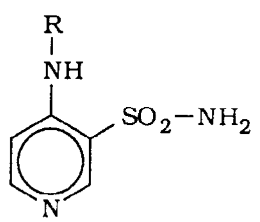

(II)

  in der R die bezüglich der Formel (I) angegebenen Bedeutungen besitzt, mit einem Isocyanat oder Isothiocyanat der Formel (III)

  $$X = C = N - R_1 \qquad \text{(III)}$$

  in der X und $R_1$ die bezüglich der Formel (I) angegebenen Bedeutungen besitzen, in Gegenwart eines alkalischen Mittels, wie eines Metallhydroxids oder eines tertiären Amins in einem polaren Lösungsmittel kondensiert, so daß man nach der Neutralisation des Reaktionsmediums, der gewünschtenfalls ein Verdampfen des Lösungsmittels und eine Wiederaufnahme in Wasser vorausgeht, die Derivate der Formel (I) erhält, die gegebenenfalls durch Zugabe einer anorganischen oder organischen, pharmazeutisch annehmbaren Säure in ihre Salze überführt werden,

  oder die man gewünschtenfalls in ihre Isomeren auftrennt und gegebenenfalls durch Zugabe einer anorganischen oder organischen, pharmazeutisch annehmbaren Säure in ihre Salze überführt,
- oder ein Derivat der Formel (IV)

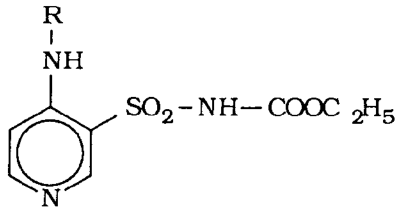

(IV)

in der R die bezüglich der Formel (I) angegebenen Bedeutungen besitzt, mit einem Amin der Formel (V)

$$R_1 - NH_2 \qquad (V)$$

in der $R_1$ die bezüglich der Formel (I) angegebenen Bedeutungen besitzt,
durch Erhitzen in einem wasserfreien Lösungsmittel kondensiert, so daß man nach der eventuellen Reinigung und Neutralisation die Verbindungen der Formel (I) erhält, in der X ein Sauerstoffatom bedeutet,
die anschließend gegebenenfalls mit einer anorganischen oder organischen, pharmazeutisch annehmbaren Säure in ihre Salze überführt werden,
oder die man gewünschtenfalls in ihre Isomeren auftrennt und dann gegebenenfalls durch Zugabe einer anorganischen oder organischen, pharmazeutisch annehmbaren Säure in ihre Salze überführt.

**2.** Verfahren nach Anspruch 1 zur Herstellung der Verbindung der allgemeinen Formel 1, in der R und $R_1$ eine Cycloalkyl-, Bicycloalkyl- oder Polycycloalkylgruppe mit 3 bis 15 Kohlenstoffatomen, die gegebenenfalls durch eines oder mehrere Stickstoffatome unterbrochen sind, sowie von deren Salzen, Enantiomeren und Diastereoisomeren.

**3.** Verfahren nach Anspruch 1 zur Herstellung der Verbindung der allgemeinen Formel I, nämlich N-(4-(Cyclohexylamino)-pyrid-3-yl)-sulfonyl)-N′-cyclohexylharnstoff und von dessen Salzen.

**4.** Verfahren nach Anspruch 1 zur Herstellung der Verbindung der allgemeinen Formel 1, nämlich N-((4-(Cycloheptylamino)-pyrid-3-yl)-sulfonyl)-N′-cyclohexylharnstoff und von dessen Salzen.

**5.** Verfahren nach Anspruch 1 zur Herstellung der Verbindung der allgemeinen Formel I, nämlich N-((4-(Cyclooctylamino)-pyrid-3-yl)-sulfonyl)-N′-cyclooctylharnstoff und von dessen Salzen.

**6.** Verfahren nach Anspruch 1 zur Herstellung der Verbindung der allgemeinen Formel I, nämlich N-((4-(Cycloheptylamino)-pyrid-3-yl)-sulfonyl)-N′-cyclohexylthioharnstoff und von dessen Salzen.

**7.** Verfahren nach Anspruch 1 zur Herstellung der Verbindung der allgemeinen Formel I, nämlich N-((4-(Norbornylamino)-pyrid-3-yl)-sulfonyl)-N′-diisopropylthioharnstoff und von dessen Salzen.

**8.** Verfahren nach Anspruch 1 zur Herstellung der Verbindung der allgemeinen Formel I, nämlich N-((4-(Cyclooctylamino)-pyrid-3-yl)-sulfonyl)-N′-ethylthioharnstoff und von dessen Salzen.

**9.** Verfahren nach Anspruch 1 zur Herstellung der Verbindung der allgemeinen Formel I, nämlich N-((4-(Cyclododecylamino)-pyrid-3-yl)-sulfonyl)-N′-ethylharnstoff und von dessen Salzen.

**10.** Verfahren nach Anspruch 1 zur Herstellung der Verbindung der allgemeinen Formel I, nämlich N-((4-(2-(Aza-2-bicyclo[3.3.0]octyl)-amino)-pyrid-3-yl)-sulfonyl)-N′-isopropylharnstoff, von dessen Salzen und dessen Diastereoisomeren.

**11.** Verfahren zur Herstellung von pharmazeutischen Zubereitungen enthaltend als Wirkstoff mindestens eine Verbindung nach einem der Ansprüche 1 bis 10 allein oder in Kombination mit einem oder mehreren inerten, nichttoxischen Trägermaterialien.

**12.** Verfahren nach Anspruch 1 1 zur Herstellung von pharmazeutischen Zubereitungen, die zur Behandlung der arteriellen Hypertension und von ödematösen Zuständen geeignet sind.

## Claims

### Claims for the following Contracting States : AT, BE, CH, DE, DK, FR, GB, IT, LI, LU, NL, SE

1. Compounds of formula (I)

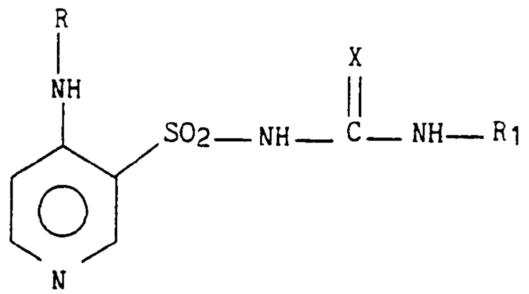

(I)

in which:
- R represents a cycloalkyl, bicycloalkyl or polycycloalkyl radical having from 3 to 15 carbon atoms optionally interrupted by one or more hetero atoms selected from nitrogen, oxygen and sulphur,
- X represents an oxygen atom or a sulphur atom, and
- $R_1$ represents a straight- or branched-chained alkyl radical having from 1 to 6 carbon atoms, or a cycloalkyl, bicycloalkyl or polycycloalkyl radical containing from 3 to 15 carbon atoms optionally interrupted by one or more hetero atoms selected from nitrogen, oxygen and sulphur,
  with the proviso that when X is an oxygen atom and R is at the same time a monocyclic or bicyclic alkyl radical, $R_1$ may not represent a linear or branched alkyl radical or a saturated nitrogen-containing heteromonocyclic group that may contain a second hetero atom,

and their addition salts with a pharmaceutically acceptable mineral or organic acid,
and, when R or $R_1$ represents a radical having one or more centers of asymmetry, their isomers, enantiomers and diastereoisomers.

2. Compounds according to claim 1 in which R and $R_1$ represent a cycloalkyl, bicycloalkyl or polycycloalkyl radical having from 3 to 15 carbon atoms optionally interrupted by one or more nitrogen atoms, and their salts, enantiomers and diastereoisomers.

3. N-((4-(cyclohexylamino)pyrid-3-yl)sulphonyl)-N'-cyclohexylurea and its salts.

4. N-((4-(cycloheptylamino)pyrid-3-yl)sulphonyl)-N'-cyclohexylurea and its salts.

5. N-((4-(cyclooctylamino)pyrid-3-yl)sulphonyl)-N'-cyclooctylurea and its salts.

6. N-((4-(cycloheptylamino)pyrid-3-yl)sulphonyl)-N'-cyclohexylthiourea and its salts.

7. N-((4-(norbornylamino)pyrid-3-yl)sulphonyl)-N'-isopropylthiourea and its salts.

8. N-((4-(cyclooctylamino)pyrid-3-yl)sulphonyl)-N'-ethylthiourea and its salts.

9. N-((4-(cyclododecylamino)pyrid-3-yl)sulphonyl)-N'-ethylurea and its salts.

10. N-((4-((2-aza-2-bicyclo[3.3.0]octyl)amino)pyrid-3-yl)sulphonyl)-N'-isopropylurea, its salts and its diastereoisomers.

11. Process for the preparation of compounds of formula (I), characterised in that:
    - either a compound of formula (II)

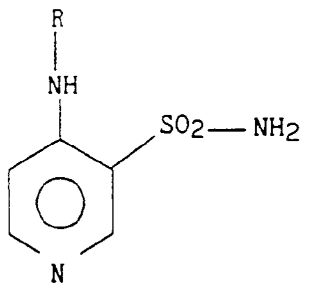

(II),

in which R has the same meaning as in formula (I), is condensed with an isocyanate or isothiocyanate of formula (III)

$$X = C = N - R_1 \qquad (III),$$

in which X and $R_1$ have the same meaning as in formula (I),
in the presence of an alkaline agent, such as a metal hydroxide or a tertiary amine, in a polar solvent, to yield, after neutralisation of the reaction mixture preceded, if desired, by evaporation of the solvent and taking up in water, the compounds of formula (I) which are optionally converted into salts by the addition of a pharmaceutically acceptable mineral or organic acid,
or, if desired, are separated into their isomers and then optionally converted into salts by the addition of a pharmaceutically acceptable mineral or organic acid,

- or a compound of formula (IV)

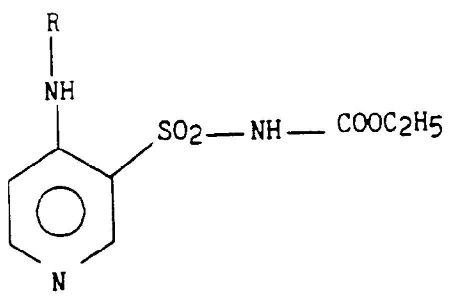

(IV),

in which R has the same meaning as in formula (I), is condensed with an amine of formula (V)

$$R_1 - NH_2 \qquad (V),$$

in which $R_1$ has the same meaning as in formula (I), by heating in an anhydrous solvent to obtain, optionally after purification and neutralisation, the compounds of formula (I) in which X represents an oxygen atom,
which compounds are then optionally converted into salts with a pharmaceutically acceptable mineral or organic acid,
or, if desired, are separated into their isomers and then optionally converted into salts by the addition of a pharmaceutically acceptable mineral or organic acid.

**12.** Pharmaceutical compositions containing as active ingredient at least one compound according to any one of claims 1 to 10, on its own or in combination with one or more inert, non-toxic excipients.

**13.** Pharmaceutical compositions according to claim 12 for use in the treatment of arterial hypertension and of oedematous conditions.

**Claims for the following Contracting States : ES, GR**

**1.** Process for the preparation of compounds of formula (I)

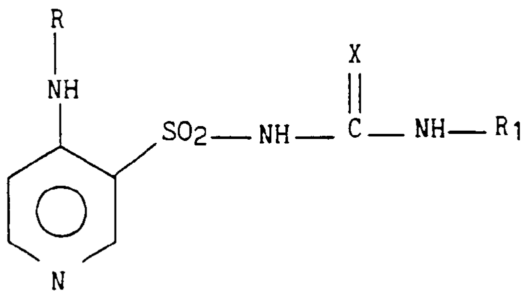

(I)

in which:

- R represents a cycloalkyl, bicycloalkyl or polycycloalkyl radical having from 3 to 15 carbon atoms optionally interrupted by one or more hetero atoms selected from nitrogen, oxygen and sulphur,
- X represents an oxygen atom or a sulphur atom, and
- $R_1$ represents a straight- or branched-chained alkyl radical having from 1 to 6 carbon atoms, or a cycloalkyl, bicycloalkyl or polycycloalkyl radical containing from 3 to 15 carbon atoms optionally interrupted by one or more hetero atoms selected from nitrogen, oxygen and sulphur, with the proviso that when X is an oxygen atom and R is at the same time a monocyclic or bicyclic alkyl radical, $R_1$ may not represent a linear or branched alkyl radical or a saturated nitrogen-containing heteromonocyclic group that may contain a second hetero atom,

and their addition salts with a pharmaceutically acceptable mineral or organic acid,
and, when R or $R_1$ represents a radical having one or more centers of asymmetry, their isomers, enantiomers and diastereoisomers,
characterised in that:

- either a compound of formula (II)

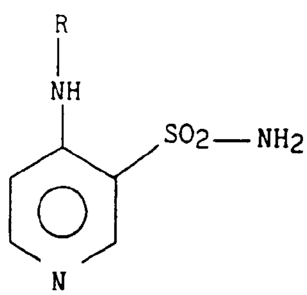

(II),

in which R has the same meaning as in formula (I), is condensed with an isocyanate or isothiocyanate of formula (III)

$$X = C = N - R_1 \qquad (III),$$

in which X and $R_1$ have the same meaning as in formula (I),
in the presence of an alkaline agent, such as a metal hydroxide or a tertiary amine, in a polar solvent, to yield, after neutralisation of the reaction mixture preceded, if desired, by evaporation of the solvent and taking up in water, the compounds of formula (I) which are optionally converted into salts by the addition of a pharmaceutically acceptable mineral or organic acid,
or, if desired, are separated into their isomers and then optionally converted into salts by the addition of a pharmaceutically acceptable mineral or organic acid,
- or a compound of formula (IV)

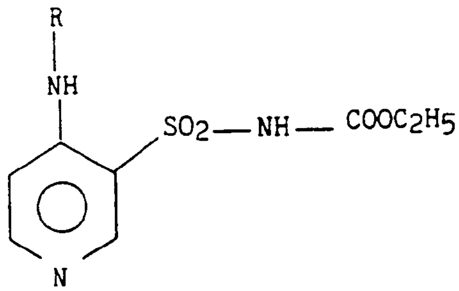

(IV),

in which R has the same meaning as in formula (I), is condensed with an amine of formula (V)

$R_1 - NH_2$ (V),

in which $R_1$ has the same meaning as in formula (I), by heating in an anhydrous solvent to obtain, optionally after purification and neutralisation, the compounds of formula (I) in which X represents an oxygen atom,
which compounds are then optionally converted into salts with a pharmaceutically acceptable mineral or organic acid,
or, if desired, are separated into their isomers and then optionally converted into salts by the addition of a pharmaceutically acceptable mineral or organic acid.

2. Process according to claim 1 for the preparation of compounds of the general formula (I) in which R and $R_1$ represent a cycloalkyl, bicycloalkyl or polycycloalkyl radical having from 3 to 15 carbon atoms optionally interrupted by one or more nitrogen atoms, and their salts, enantiomers and diastereoisomers.

3. Process according to claim 1 for the preparation of a compound of the general formula (I) which is N-((4-(cyclohexylamino)pyrid-3-yl)sulphonyl)-N'-cyclohexylurea, and its salts.

4. Process according to claim 1 for the preparation of a compound of the general formula (I) which is N-((4-(cycloheptylamino)pyrid-3-yl)sulphonyl)-N'-cyclohexylurea, and its salts.

5. Process according to claim 1 for the preparation of a compound of the general formula (I) which is N-((4-(cyclooctylamino)pyrid-3-yl)sulphonyl)-N'-cyclooctylurea, and its salts.

6. Process according to claim 1 for the preparation of a compound of the general formula (I) which is N-((4-(cycloheptylamino)pyrid-3-yl)sulphonyl)-N'-cyclohexylthiourea, and its salts.

7. Process according to claim 1 for the preparation of a compound of the general formula (I) which is N-((4-(norbornylamino)pyrid-3-yl)sulphonyl)-N'-isopropylthiourea, and its salts.

8. Process according to claim 1 for the preparation of a compound of the general formula (I) which is N-((4-(cyclooctylamino)pyrid-3-yl)sulphonyl)-N'-ethylthiourea, and its salts.

9. Process according to claim 1 for the preparation of a compound of the general formula (I) which is N-((4-(cyclododecylamino)pyrid-3-yl)sulphonyl)-N'-ethylurea, and its salts.

10. Process according to claim 1 for the preparation of a compound of the general formula (I) which is N-((4-((2-aza-2-bicyclo[3.3.0]octyl)amino)pyrid-3-yl)sulphonyl)-N'-isopropylurea, its salts and its diastereoisomers.

11. Process for the preparation of pharmaceutical compositions containing as active ingredient at least one compound according to any one of claims 1 to 10, on its own or in combination with one or more inert, non-toxic excipients.

12. Process according to claim 11 for the preparation of pharmaceutical compositions according to claim 11 for use in the treatment of arterial hypertension and of oedematous conditions.